# EUROPEAN PATENT APPLICATION

(11) **EP 4 793 926 A1**
(43) Date of publication of application: **19.08.2026**
(21) Application number: 24875641.3
(22) Date of filing: 09.10.2024
(51) Int. Cl.: G09B 23/28, G09B 19/00

(54) **CIRCULATION LIQUID AND LUBRICITY ADJUSTING AGENT**

(30) Priority: 10.10.2023 JP 2023175599
(71) Applicant: Fain-Biomedical Inc., Nagoya-shi, Aichi 451-0042 (JP)
(72) Inventor: IKEDA, Seiichi, Okayama-shi, Okayama 701-1221 (JP); IKEDA, Moritaka, Okayama-shi, Okayama 701-1221 (JP)
(74) Representative: Algemeen Octrooi- en Merkenbureau B.V.
(86) International application number: PCT/JP2024/036207
(87) International publication number: WO 2025/079625

(57) **Abstract**

Even in a case where a water-based circulating liquid is applied to a blood vessel model, achieved is a lubricating property that can withstand a pressing force equivalent to that during insertion of a catheter into a real human blood vessel.

A circulating liquid used in a catheter simulator, the circulating liquid comprising a water-soluble polymer, in which the water-soluble polymer includes a water-soluble polymer having both a hydrophilic group and a hydrophobic group, and/or a mixture of a polymer having a hydrophilic group and a polymer having a hydrophobic group.

## Description

### TECHNICAL FIELD

The present invention is suitable for use in a circulating liquid for a catheter simulator using a blood vessel model made of silicone rubber, urethane rubber, or the like. In addition, the lubricity adjusting agent of the present invention improves lubricity between cross linkable polymer materials.

### BACKGROUND ART

The present inventor has developed and put on the market a catheter simulator that simulates a human body (see Patent Literature 1). In this catheter simulator, a partition member is incorporated in a mannequin's main body made of a transparent material, a blood vessel model as a stereoscopic model is supported on one surface of the partition member, and an auxiliary tool for causing the blood vessel model to operate is disposed. The blood vessel model is formed of silicone rubber and the auxiliary tool includes a tank, a pump, and a connecting tube. In the tank, a circulating liquid is stored, and the circulating liquid is circulated in the blood vessel model through the connecting tube by the pump. In a case where the catheter is inserted into the blood vessel model, lubricity between a surface of the silicone rubber and a surface of the catheter becomes a problem.

In addition, Patent Literature 2 is referred to as a literature disclosing a technique related to the present invention.

### CITATIONS LIST

### PATENT LITERATURE

Patent Literature 1: JP 2006-267565 A
Patent Literature 2: JP 5992031 B2

### SUMMARY OF INVENTION

### TECHNICAL PROBLEMS

According to the catheter simulator disclosed in Patent Literature 1, the catheter can be inserted into the blood vessel model while the circulating liquid is caused to flow through the blood vessel model made of silicone rubber.

As the circulating liquid, one mainly containing a silicone oil (an oil-based circulating liquid) and one mainly containing water (a water-based circulating liquid) are used, but the water-based circulating liquid is preferable from a viewpoint of similarity with blood. In a case where water is simply circulated in the blood vessel model, contact resistance of the catheter against an inner wall of the blood vessel model increases, and the catheter cannot be smoothly inserted thereinto.

Therefore, a surfactant is mixed with water as a lubricity adjusting agent. Accordingly, frictional resistance between the catheter and an inner peripheral surface of the blood vessel model is reduced, and the catheter can be smoothly inserted into the blood vessel model.

However, even if the circulating liquid obtained by mixing the surfactant with the water is circulated in the blood vessel model, real blood circulating blood vessel is not imitated. Accordingly, in the simulation using such circulating liquid, it cannot be denied that there is a feeling of strangeness in a sensation of inserting the catheter as compared with that during actual surgery. For example, when the catheter passes through a portion where the blood vessel model meanders or the like, the frictional resistance has been extremely larger than that during the actual surgery. In addition, if the inserted catheter is left to stand for several seconds, a coefficient of static friction between the catheter and the blood vessel model becomes abnormally large, and phenomena (hereinafter, these phenomena are referred to as "adhesion") in which it is difficult to push and pull the inserted catheter have been observed.

The lubricity adjusting agent disclosed in Patent Literature 2 has been made to solve this problem, and using the surfactant and a water-soluble metal salt in combination as the lubricity adjusting agent can bring the sensation of inserting the catheter into the blood vessel model close to the sensation of inserting the catheter during the actual surgery. If the lubricity adjusting agent disclosed in Patent Literature 2 is used, the adhesion between the blood vessel model and the catheter can be prevented even at a site of the meandered blood vessel model, and the problem that insertion resistance suddenly increases during catheter operation can be solved.

Even in a case of using the lubricity adjusting agent disclosed in Patent Literature 2, the adhesion may occur if a force by which the catheter is pressed to a blood vessel wall (hereinafter, referred to as a "pressing force") increases to 5 N (about 0.5 kg) or more. Also in a human blood vessel, similar adhesion occurs as the pressing force increases. However, as a result of evaluation by the present inventors, it has been confirmed that the adhesion occurs due to a small pressing force as compared with that in the human blood vessel even in the case of using the lubricity adjusting agent. In order to avoid the adhesion, it has been attempted to increase the mixed amount of the surfactant or the water-soluble ionic compound, but the effect thereof is saturated even in a case where the added amount is increased, and the same property as that of the human blood vessel was not performed. In addition, it is not preferable to increase the mixed amount of the surfactant because a slimy feeling occurs in the circulating liquid.

Use of silicone oil as the circulating liquid is effective for avoiding the adhesion. However, in a so-called oil-based silicone oil (including emulsion thereof in which the silicone oil is dispersed in water with an emulsifier or the like), or a water-soluble silicone oil such as a polyether-modified silicone, a handling sensation during the catheter insertion lacked reality due to significant differences in physical properties compared with blood.

### SOLUTIONS TO PROBLEMS

The present inventor has conducted intensive studies in order to achieve a lubricating property that can withstand a pressing force equivalent to that during the insertion of the catheter into the real human blood vessel also in a case where a so-called water-based circulating liquid is applied to the blood vessel model. As a result, it has been found that by using a specific water-soluble polymer, the adhesion resistance property of the catheter against the blood vessel model is remarkably enhanced, and is close to that of the human blood vessel during the actual surgery. As a result, even in a region such as iliac artery region, aorta region,, or a common carotid artery region where a large pressing force is likely to be generated during surgery, the inner peripheral surface of the blood vessel model and the catheter did not adhere to each other, and the catheter can be passed with a similar operation sensation as in the actual surgery.

In other words, a first aspect of the present invention is defined as follows.

A circulating liquid used in a catheter simulator, the circulating liquid including a water-soluble polymer having both a hydrophilic group and a hydrophobic group, and/or a mixture of a polymer having a hydrophilic group and a polymer having a hydrophobic group.

According to the circulating liquid of the first aspect defined as above, in a case of the water-soluble polymer having both the hydrophilic group and the hydrophobic group, the hydrophobic group (a lipophilic group) of the water-soluble polymer is linked to an inner peripheral surface of a blood vessel model, thereby its hydrophilic group appears on the outside. The hydrophilic group of the water-soluble polymer is linked to the appeared hydrophilic group, thereby its hydrophobic group appears on the outside. The hydrophobic group of the water-soluble polymer is linked to the appeared hydrophobic group, and its hydrophilic group appears on the outside as well. In this way, several layers of the water-soluble polymer overlapping on the inner peripheral surface of the blood vessel model are formed. Although a bonding force between the layers of such water-soluble polymer is loose, for example, a polymer chain having a certain length is complicatedly entangled, so that a tough lubricating layer is formed, and it is difficult to separate the tough lubricating layer from the inner peripheral surface. Accordingly, the inner peripheral surface of the blood vessel model has physical properties similar to those of endothelial cells existing inside the human blood vessel, and the lubricity can be maintained without causing the adhesion even in a case where a large pressing force is applied to the catheter. As a result, even in a region such as a cerebral artery where a normally small pressing force is applied, or even in a region such as an aorta where a large pressing force is applied, it was possible to demonstrate a similar passing ability and operation sensation (friction feeling) of the catheter as in the actual surgery.

By subjecting the inner surface of the blood vessel model to a hydrophilization treatment in advance by various methods, it was also possible to show the lubricating property close to that of the human blood vessel. However, in the hydrophilic layer formed in this way, the property was likely to change due to degradation over time, adhesion of contaminants to the surface, abrasion in the use process, and the like. According to the method of the present invention, even in a case where the water-soluble polymer adhering to the inner peripheral surface of the blood vessel model is lost due to the abrasion or the like, the water-soluble polymer dissolved in the circulating liquid adheres to the inner peripheral surface of the blood vessel model again for replenishment, so that certain properties can be continuously maintained. In addition, after being used, the water-soluble polymer and the like are discharged and removed together with the circulating liquid, and are replenished again together with a new circulating liquid when being used at the next time, so that a new water-soluble polymer is always supplied, and the properties do not vary.

As a result, in the catheter simulator including the blood vessel model in which the circulating liquid of the first aspect is circulated, the insertion sensation when the catheter is inserted into the blood vessel model becomes close to the sensation of inserting the catheter into a human blood vessel, and the adhesion resistance property is maintained even in a case where a large pressing force is applied to the catheter due to the multiply-overlapped layers of the water-soluble polymer.

Here, examples of the water-soluble polymer having both the hydrophilic group and the hydrophobic group can include polyvinyl alcohol (PVA) and methyl cellulose.

In addition to natural polymers such as proteins and starches, a synthetic polymer such as polyacrylic acid, polyacrylamide, polyethylene oxide, poly (vinyl pyrrolidone), polyvinyl amide, or polyamine can be included.

The molecular weights (polymerization degrees) and the blended amounts of these water-soluble polymers can be arbitrarily selected according to a diameter and an area of the inner peripheral surface of the blood vessel model used in the catheter simulator, the type of the catheter, and the like.

The blended amount of the water-soluble polymer is preferably about 0.5% to about 8.0% at a mass ratio with respect to water.

PVA is preferably used from a viewpoint of cost, decomposability after use, cleanability, and the like of a material.

In the case of PVA, the average molecular weight (number average) thereof is preferably about 500 to about 2000, and the saponification degree thereof is preferably 75 or more.

In the case of PVA, from a viewpoint of ensuring the dispersibility thereof and from a viewpoint of bringing the sensation close to that of human blood, it is preferable to maintain the pH in a range from weak basicity to weak acidity, and for this purpose, it is preferable to comprise a pH buffer therewith.

Since PVA is a so-called water-based glue, a film is formed and adheres to various base materials when moisture evaporates after use of the catheter simulator, and clogging, sticking, or the like may occur to interfere with subsequent use. In order to prevent the formation of the film of the water-soluble polymer such as PVA, it is preferable to mix a saccharide (sucrose, fructose, or the like) and the like in the circulating liquid. By mixing the saccharide and the like, when the circulating liquid containing the water-soluble polymer such as PVA dries (evaporates), the formation of the film is suppressed, the circulating liquid becomes powdered after the drying, and becomes easily removed even in a case where the circulating liquid adheres to various base materials.

The blended amount of the saccharide can be appropriately selected according to the properties of the water-soluble polymer such as PVA, but it is preferable to blend 50 to 200 parts by mass of the saccharide with respect to 100 parts by mass of PVA.

In a case of a mixture of the water-soluble polymer having the hydrophilic group and the polymer having the hydrophobic group, it can be considered that both the polymers are entangled with each other in the circulating liquid, and apparently form a molecule having the hydrophilic group and the hydrophobic group. Such molecule having both the hydrophilic group and the hydrophobic group is singly overlapped several times on the inner peripheral surface of the blood vessel model similarly to the above-described water-soluble polymer having both the hydrophilic group and the hydrophobic group such as the PVA or the methyl cellulose, to form a layer of the molecule exhibiting the lubricity and the toughness similar to those of PVA and methyl cellulose.

Here, a blending ratio of the water-soluble polymer having the hydrophilic group and the polymer having the hydrophobic group is preferably 1 : 1 in terms of the total amount of the hydrophilic group and the total amount of the hydrophobic group, but is not particularly limited.

In order to reliably entangle the water-soluble polymer having the hydrophilic group with the polymer having the hydrophobic group, a certain length is required for the main chain constituting the polymers.

In addition to the water and the water-soluble polymer described above, it is preferable to add the lubricity adjusting agent introduced in Patent Literature 2 to the circulating liquid. Here, the described contents of Patent Literature 2 are incorporated herein as reference.

The water-soluble polymer having both the hydrophilic group and the hydrophobic group, and a mixture of the water-soluble polymer having the hydrophilic group and the polymer having the hydrophobic group described above are also lubricity adjusting agents.

The lubricity adjusting agent can be blended into the circulating liquid of the catheter simulator singly, together with a surfactant, or together with a surfactant and a water-soluble ionic compound.

In the lubricity adjusting agent, a role of the surfactant is mainly to reduce a coefficient of dynamic friction between objects (such as between the surface of the silicone rubber and the surface of the catheter), and to promote bonding (multilayering) of the water-soluble polymer by interaction (ionic bonding, hydrophobic bonding, hydrogen bonding, covalent bonding, and the like) between the surfactant and the water-soluble polymer. Accordingly, for example, in a case where the catheter comes into contact with a surface of a silicone rubber-made blood vessel model, the resistance during the insertion of the catheter is reduced, and an operator can smoothly insert the catheter into the blood vessel model.

On the other hand, in addition to the above reduction of the dynamic friction coefficient, a role of the water-soluble ionic compound is to prevent adhesion between the silicone rubber and a member in contact with the silicone rubber (that is, reduction in coefficient of static friction), to further promote the bonding (multilayering) of the water-soluble polymer by the interaction (the ionic bonding, the hydrophobic bonding, the hydrogen bonding, the covalent bonding, and the like) among the water-soluble ionic compound, the surfactant, and the water-soluble polymer, and to enhance the toughness of the lubricating layer formed.

In a case where a layer including the surfactant and the water-soluble ionic compound but not including the water-soluble polymer, the layer easily collapses if the pressing force of the catheter increases, and the catheter and the surface of the blood vessel model come into direct contact with each other to cause the adhesion.

Similarly to the surfactant, the water-soluble polymer having the hydrophilic group and the hydrophobic group has an effect of reducing the coefficient of dynamic friction by forming a lubricating layer between the objects (such as between the surface of the silicone rubber and the surface of the catheter). The circulating liquid obtained by further adding the water-soluble polymer to the surfactant and the water-soluble ionic compound is multilayered as a lamellar structure due to the interaction (the ionic bonding, the hydrophobic bond, the hydrogen bond, the covalent bond, and the like) between the surfactant and the water-soluble ionic compound, and molecular chains of the water-soluble polymer are complicatedly entangled in a wide range to form a tough lubricating layer. As a result, the adhesion resistance of the lubricating layer was greatly improved as compared with a case where only the surfactant and the water-soluble ionic compound were used, the adhesion did not occur even in a case where a large pressing force of 15 N (about 1.5 kg) or more was applied to the catheter, and the lubricating property similar to that of the human blood vessel can be maintained. In a case where the water-soluble polymer is not added, the lubricating layer that can withstand the pressing force of 15 N for several seconds or more was not formed.

According to the evaluation by the present inventors, the lubricating layer formed on the inner peripheral surface of the blood vessel model was remarkably tough against the pressing force of the catheter particularly in a case where three of the surfactant, the water-soluble ionic compound, and the water-soluble polymer having the hydrophilic group and the hydrophobic group coexist, and can withstand the pressing force of the catheter comparable to that of the human blood vessel.

In a real biological blood vessel, the catheter or a guide wire is designed to slide well along an inner wall of the blood vessel. On the other hand, since an aneurysm embolization coil or the like is indwelled in the body after surgery, it is necessary to stably maintain the installation state, and the aneurysm embolization coil or the like is designed not to slide (not to move) in the biological blood vessel (in an aneurysm).

In a case where only the surfactant and the water-soluble ionic compound were added as in the circulating liquid that has been conventionally used, the catheter, the guide wire, and the aneurysm embolization coil inserted into the blood vessel model were similarly improved in slipperiness according to an increase in the added amount, and a difference in lubricity improvement tendency thereamong has not been confirmed. As a result, in a case where the coil is indwelled in the aneurysm set in the blood vessel model, the coil continued to move unstably due to catheter operation during the indwelling, blood flow after the indwelling, and the like both in the indwelling process and after the indwelling unlike the actual surgery.

On the other hand, in a case where three of the surfactant, the water-soluble ionic compound, and the water-soluble polymer having the hydrophilic group and the hydrophobic group were added as in the present invention, the slipperiness of the catheter or the guide wire was improved (the friction coefficient greatly decreased) according to the increase in the added amount, but a change in the slipperiness of the aneurysm embolization coil has not been confirmed (the friction coefficient hardly changed). As a result, in the case where the aneurysm embolization coil was indwelled in the aneurysm formed in the blood vessel model, the catheter and the guide wire exhibited good slipperiness similarly in the biological blood vessel, whereas the coil hardly moved due to the catheter operation, the blood flow, or the like and can stably stay at the position both in the indwelling process and after the indwelling as during the actual surgery.

As described above, in the circulating liquid of the present invention, experimental results clearly show that a lubrication mechanism itself is different from the circulating liquid (added with only the surfactant and the water-soluble ionic compound) that has been conventionally used. As a result, by using the circulating liquid of the present invention, it was possible to distinctively adjust and show the similar friction properties of the catheter and the guide wire and the aneurysm embolization coil or the like, and to well show the similar lubricating property as that in the biological blood vessel.

Another property of the circulating liquid of the present invention is improvement in water retainability (difficulty in drying). The addition of the conventional circulating liquid (only the surfactant or the water-soluble ionic compound) has not been confirmed to significantly affect the water retainability (difficulty in drying) of a liquid (usually water), but in the circulating liquid of the present invention, a network was formed by the interaction among the surfactant, the water-soluble ionic compound, and the water-soluble polymer, and moisture was stored therein, so that the water retainability (difficulty in drying) was significantly improved.

As still another property, in the liquid (usually water based) to which the circulating liquid of the present invention was added, gas dissolved in the liquid was not deposited on a surface of an object such as a container holding the liquid, or the blood vessel model or the catheter immersed in the liquid, and even bubbles originally present were dissolved in the circulating liquid over time and disappeared. In addition, in a case where the liquid such as water is disposed in a sealed container with a gap area left (in a case of the sealed container without being liquid-tightly sealed), generally, fogging occurred on a surface of the container facing the gap area due to evaporation of the liquid. However, in a case of using the circulating liquid of the present invention, such fogging did not occur (in either a case where the circulating liquid is in contact with the surface of the container as well as not in contact with the surface). In the conventional circulating liquid, this bubble generation/adhesion phenomenon was not affected (the addition of the surfactant makes the bubbles finer or easier to adhere, but the bubble generation itself was not changed, and the effect of taking the bubbles into the liquid and eliminating the bubbles therefrom was not obtained).

Even in the case where the water based conventional circulating liquid was used, the bubbles dissolved in the circulating liquid were deposited and attached to the surface of the object, and the visibility was reduced during the catheter simulation. Therefore, it is necessary to periodically remove the bubbles by stirring work or the like. On the other hand, in the case of using the circulating liquid of the present invention, the bubbles did not occur during the simulation, the bubbles that have existed from the beginning in a preparation stage or the like and the bubbles that have flowed into the liquid through the catheter automatically disappeared, and a bubble-free state is naturally formed and maintained at all times in the liquid or on the surface of the objects. Therefore, the visibility is greatly improved, the bubble removal work became unnecessary, and surgical simulation can be performed comfortably. It can be considered that this effect can be effectively applied to applications other than the catheter surgery simulation as the circulating liquid.

In addition, in the case where three of the surfactants, the water-soluble ionic compound, and the water-soluble polymer having the hydrophilic group and the hydrophobic group were added as in the present invention, the viscosity of the circulating liquid increased. In a case where only the surfactant and the water-soluble ionic compound were added to the water as in the conventional circulating liquid, significant change has not been confirmed in the viscosity (in tap water to which the surfactant (0.4 wt%) and water-soluble ionic compound saturated water (2 wt%) were added, the viscosity was 1.28 mPa·s with respect to the viscosity of the tap water of 1.25 mPa·s). However, in a case where the water-soluble polymer (PVA, 1.1 wt%) was further added thereto, the viscosity greatly increased (2.91 mPa·s), and the viscosity largely deviated from the viscosity of biological blood (about 1.3 to 1.7 mPa·s). As a result, in a case where the catheter was inserted into the blood vessel model and a pressure corresponding to a blood pressure (120 mmHg) was applied to the circulating fluid filling the blood vessel model, there was also a change in a flow rate or the like that passed through the inside of the catheter and flowed out from another end of the catheter (in a case of using an excelsior 1018 microcatheter to which a pressure of 120 mmHg was applied, the outflow amount in the case of using the conventional circulating liquid was 0.83 ml/min, and the outflow amount in the case of using the circulating liquid of the present invention was 0.33 ml/min).

As a result of intensive studies on a method for lowering the viscosity to a value similar to that of blood while maintaining excellent lubricating property in the case where three of the surfactant, the water-soluble ionic compound, and the water-soluble polymer having the hydrophilic group and the hydrophobic group were added as in the present invention, it has been found that the viscosity can be adjusted by further adding a pH adjusting agent (a buffer material) and changing the pH. Even in the case of using the circulating liquid of the present invention, the viscosity similar to that of blood can be showed without impairing the excellent lubricating property (when no pH adjusting agent was added, the pH was 6.1, and the viscosity was 2.91 mPa·s, whereas when the pH adjusting agent (carbonate ion or the like) was added, the pH was 6.8, and the viscosity was 1.44 mPa·s). This is presumed to be caused by a change in a polymer network constituted by the surfactant, the water-soluble ionic compound, and the water-soluble polymer having the hydrophilic group and the hydrophobic group due to the addition of the pH adjusting agent, and it has been found that the pH adjusting agent can be effectively used for the purpose of adjusting the polymer network formed when the circulating liquid of the present invention is used.

Here, as the surfactant, one or two or more of the group consisting of a cationic surfactant, an anionic surfactant, a nonionic surfactant, and a zwitterionic surfactant can be appropriately used depending on a material of a sheath of the catheter, and the like.

In the above, deionized water is preferably used as water, but tap water can be used.

The type of the surfactant is preferably selected depending on the material of the catheter. For example, when the sheath is formed of Teflon (registered trademark) or polyethylene which is likely to be negatively charged when rubbed, it is preferable to use the anionic surfactant. On the other hand, when the sheath is formed of a material such as polyamide that is likely to be positively charged, it is preferable to use the zwitterionic surfactant.

It is difficult to specify a charging tendency of the material of the sheath or the like of the catheter to be used (an influence of zeta potential, charging series during frictional charging, or the like) at a scene where intravascular treatment training is performed. In a case of using tap water, components and the amount of an electrolyte are not constant, and the pH varies accordingly, so that it is more difficult to specify the charge tendency. Therefore, it is preferable to appropriately adjust the type and amount of the surfactant to be added before or during the simulation by confirming the properties during actual use, and so on. As the surfactant, the zwitterionic surfactant singly, a surfactant obtained by adding the zwitterionic surfactant to the cationic surfactant, or a surfactant obtained by adding the zwitterionic surfactant to the anionic surfactant can be used. Accordingly, regardless of the material of the sheath of the catheter, the surfactant is stably adsorbed on the surface of the sheath of the catheter.

As a blending ratio of the anionic surfactant (or the cationic surfactant) and the zwitterionic surfactant, it is preferable to blend 1 to 100 parts by mass of the zwitterionic surfactant with respect to 100 parts by mass of the anionic surfactant (or the cationic surfactant).

The concentration of the surfactant may be appropriately adjusted depending on the type of the surfactant, the type and concentration of other solvents, the intended use, and the like, but is preferably 0.005 mmol/L or more and 100 mmol/L or less. In this range, small contact resistance is secured, and preferable physical properties as the circulating liquid can be obtained (the slimy feeling cannot be obtained). The concentration is more preferably 0.05 mmol/L or more and 10 mmol/L or less.

As the water-soluble ionic compound, a water-soluble metal salt, a water-soluble ammonium salt (for example, ammonium chloride or ammonium sulfate), or the like can be used. As the water-soluble metal salt, one or two or more of the group consisting of an alkali metal salt, an alkaline earth metal salt, an aluminum salt, and an iron salt can be used.

Examples of the water-soluble alkali metal salt include sodium chloride, potassium chloride, cesium chloride, sodium sulfate, potassium sulfate, cesium sulfate, sodium nitrate, potassium nitrate, and cesium nitrate. In addition, examples of the water-soluble alkaline earth metal salt include magnesium chloride, calcium chloride, barium chloride, magnesium nitrate, calcium nitrate, and barium nitrate. Further, examples of the aluminum salt include aluminum chloride, aluminum sulfate, and aluminum nitrate. In addition, examples of the iron salt include ferrous chloride, ferric chloride, ferrous sulfate, ferric sulfate, ferrous nitrate, and ferric nitrate. In addition to the above, it is also possible to use a water-soluble alkali metal salt, the alkaline earth metal salt, a metal organic acid salt (for example, sodium acetate), and complexes thereof.

The concentration of the water-soluble metal salt in the circulating liquid is preferably 1 mmol/L or more and 100 mmol/L or less. The concentration is more preferably 2 mmol/L or more and 50 mmol/L or less.

Although the functions by the actions of these water-soluble ionic compounds are not clear, in a case where a large pressing force of 5 N (about 0.5 kg) or more is applied to the catheter in the absence of the water-soluble ionic compound, the degree of sticking (the adhesion) of the catheter to the inner peripheral surface of the blood vessel model increases.

As the water-soluble polymer having the hydrophilic group and the hydrophobic group, those having both the hydrophilic group and the hydrophobic group on the structure such as the polyvinyl alcohol and the methyl cellulose, or complexes thereof can be used.

In addition, a similar effect was obtained by mixing and using a water-soluble polymer material such as sodium polyacrylate or carboxymethyl cellulose having only the hydrophilic group, and a water-soluble polymer material such as an alginic acid having only the hydrophobic group. In a case where the water-soluble polymer material (for example, the sodium polyacrylate or the carboxymethyl cellulose) having only the hydrophilic group or the water-soluble polymer material (for example, the alginic acid) having only the hydrophobic group was singly used, the effects of the present invention were not observed.

This can be considered to be caused by the entanglement of both the materials when dispersed in the water, and the behavior of the materials as a molecule having both the hydrophilic group and the hydrophobic group in appearance.

### BRIEF DESCRIPTION OF DRAWINGS

FIG. 1 is a perspective view of a measurement apparatus that measures properties of the circulating liquid of the present invention.
FIG. 2 illustrates a sinusoidal wave trajectory of reciprocation of a catheter by the measurement apparatus.
FIG. 3 illustrates a change in a reaction force on the catheter when the catheter is reciprocated as illustrated in FIG. 2.
FIG. 4 illustrates a rectangular wave trajectory of the reciprocation of the catheter by the measurement apparatus.
FIG. 5 illustrates a change in a reaction force on the catheter when the catheter is reciprocated as illustrated in FIG. 4 in comparison with a reciprocation speed.
FIG. 6 illustrates a change in the reaction force on the catheter when the catheter is reciprocated as illustrated in FIG. 4 with respect to various circulating liquids.
FIG. 7 illustrates a change in the reaction force on the catheter when the catheter is reciprocated as illustrated in FIG. 4 with respect to various circulating liquids, with the concentrations of a surfactant and a water-soluble ionic compound kept constant.
FIG. 8 illustrates that the reaction force on the catheter is maintained even in a circulating liquid in which a saccharide is blended.

### DESCRIPTION OF EMBODIMENT

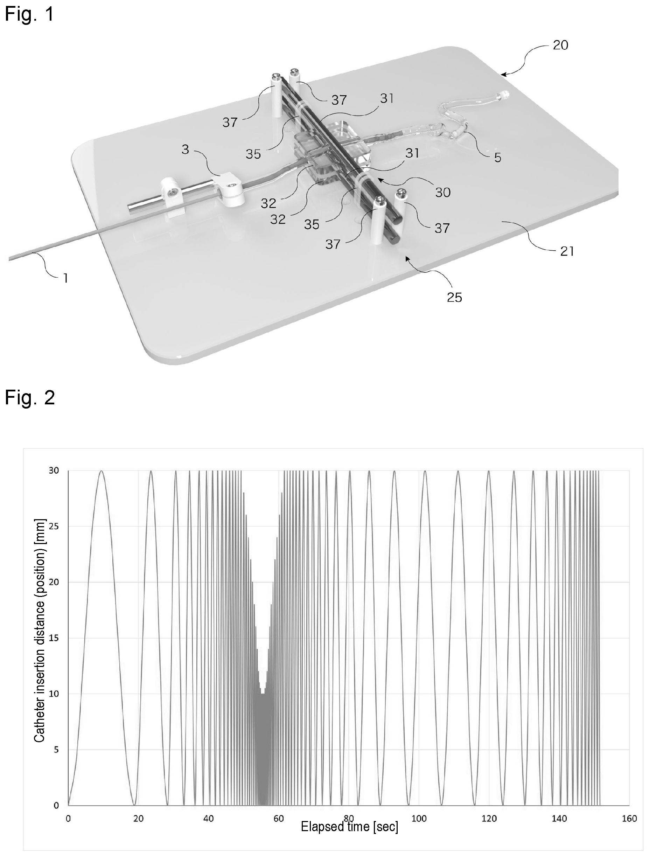

In order to measure a difference in lubricity, the present inventors made a measurement apparatus 20 of FIG. 1.

The measurement apparatus 20 includes a substrate 21, a fixing portion 25, and a clip portion 30.

Two pairs of poles 37 and 37 are erected as the fixing portions 25 on the substrate 21 made of plastic. The clip portion 30 includes a pair of bars 31 and 31 and a pair of elastic sheets 32 and 32.

The pair of bars 31 and 31 fixed by the fixing portion 25, is biased to be close to each other by rubber bands 35 and 35 when clamping a blood vessel model 5. The elastic sheets 32 and 32 are interposed between the bars 31 and 31 and the blood vessel model 5. The elastic sheets 32 and 32 are set to have a thickness of 6 mm and a hardness of HC 12.5 so as to simulate a human aortic wall. A catheter 1 is pressed to an inner peripheral surface of the blood vessel model 5 via the bars 31 and 31 and the elastic sheets 32 and 32 by a fastening force generated by the rubber bands 35 and 35, whereby the pressing force is generated between the catheter and the inner peripheral surface of the blood vessel model.

The substrate 21 made of plastic is fixed to a workpiece mounting surface of a general-purpose machine tool. By causing the machine tool to operate, the substrate 21 moves integrally with the workpiece mounting surface. Accordingly, the entire measurement apparatus 20 except the catheter 1 moves in an optional direction at an optional speed in response to an operation set by the machine tool. The illustrated left end of the catheter 1 inserted into the blood vessel model 5 is connected to a force sensor, and the force sensor is fixed to the body of the machine tool or an installation base of the body of the machine tool or the like so as to make a movement relative to the mounting surface. Note that, in the present example, the substrate 21 is fixed to the workpiece mounting surface of the machine tool to move the measurement apparatus 20 except the catheter 1. On the other hand, similar measurement can be performed by a method of fixing the force sensor connected to the catheter 1 to the workpiece mounting surface of the machine tool and fixing the measurement apparatus 20 except the catheter 1 in a space and moving the force sensor (that is, the catheter 1). In a case where a video camera or the like is installed so as to capture the fixing portion 25 or the like at a fixed point, in the configuration of the present example, the video camera may be fixed to the workpiece mounting surface via a fixing tool such as a camera arm in a manner similar to the measurement apparatus 20.

In this example, the entire measurement apparatus 20 except the catheter 1 is reciprocated in a direction perpendicular to the bars 31 and 31 (that is, in an axial direction of the catheter 1). The catheter 1 is fixed to a movable portion of a linear motion slider 3 attached to the substrate 21, and can operate only in one direction. Accordingly, the catheter 1 and the entire measurement apparatus 20 can reliably and stably make relative linear movement.

In addition, by sandwiching the blood vessel model 5 between the bars 31 and 31 via the elastic sheets 32 and 32, it is possible to provide a state similar to that where a human blood vessel is flexibly supported from the surrounding by the surrounding tissue in a living body and a pressing force of the catheter is widely dispersed in the surrounding tissue, and it is possible to obtain dynamics and measurement results of the catheter and the blood vessel close to those during actual catheter surgery.

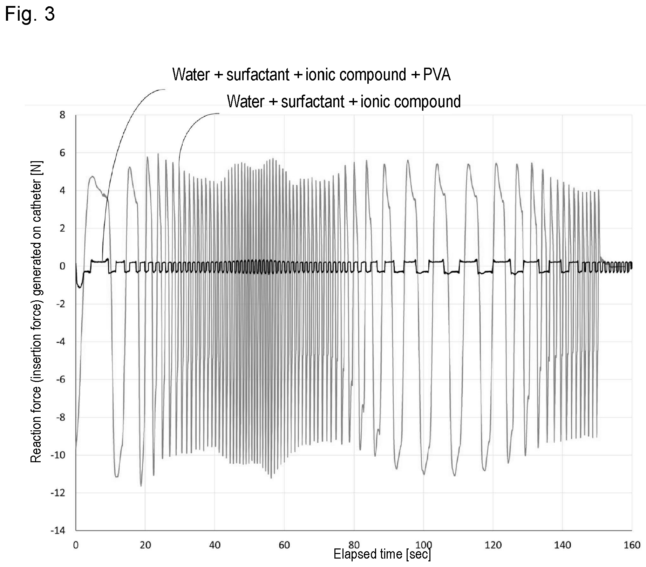

The circulating liquid of the present invention is prepared by dissolving PVA as a water-soluble polymer having a hydrophilic group and a hydrophobic group in a circulating liquid using the lubricity adjusting agent introduced in Patent Literature 2 (a circulating liquid of a comparative example), that is, a circulating liquid obtained by dissolving a surfactant and a water-soluble ionic compound in water. FIG. 3 illustrates the result measured by filling such circulating liquid in a blood vessel model made of silicone rubber, inserting a catheter having a polyethylene sheath into the blood vessel model, and then giving a sinusoidal wave-shaped reciprocation trajectory A illustrated in FIG. 2 to the measurement apparatus. The reciprocation trajectory A is set to similarly perform "an operation/dither of slightly vibrating the catheter at substantially the same position", as an operation during which an adhesion phenomenon is likely to occur (the lubricating layer is likely to be lost) during the actual surgery. A section having a small amplitude provided in the middle of the reciprocating motion trajectory A is a section particularly intended for this operation. A horizontal axis in FIG. 2 indicates time, and it can be seen from a change in frequency of the graph that the speed of the reciprocation by the arm sequentially increases from a slow speed and then decreases again.

Here, the measurement was performed in a state where 15 N (about 1.5 kg) was applied as a maximum pressing force between the catheter and a blood vessel wall surface that may be applied to the catheter during the actual surgery by adjusting the tension of the rubber bands.

As illustrated in FIG. 3, in the state where a large pressing force of 15 N is applied, a very large reaction force exceeding 10 N is generated on the catheter during the reciprocation when the circulating liquid is not containing the water-soluble polymer (containing only the surfactant and the water-soluble ionic compound). In a case of using this circulating liquid, the lubricating layer is lost and the adhesion occurs in the entire measurement section, and the reciprocation is performed in the adhesion state. Such adhesion occurred when the pressing force exceeds 5 N when using the circulating liquid. On the other hand, in the circulating liquid in which the water-soluble polymer was added to the surfactant and the water-soluble ionic compound, the lubricating layer was maintained without being lost in the entire section of the measurement, and the reciprocation was performed in the entire section while the lubricity was maintained as that in the human blood vessel.

From the results of FIG. 3, it can be seen that by blending PVA, which is a water-soluble polymer, whereby a tough lubricating layer having excellent lubricity is formed, and frictional resistance between the catheter and the blood vessel model is remarkably suppressed even in a state where a large pressing force to the catheter is applied.

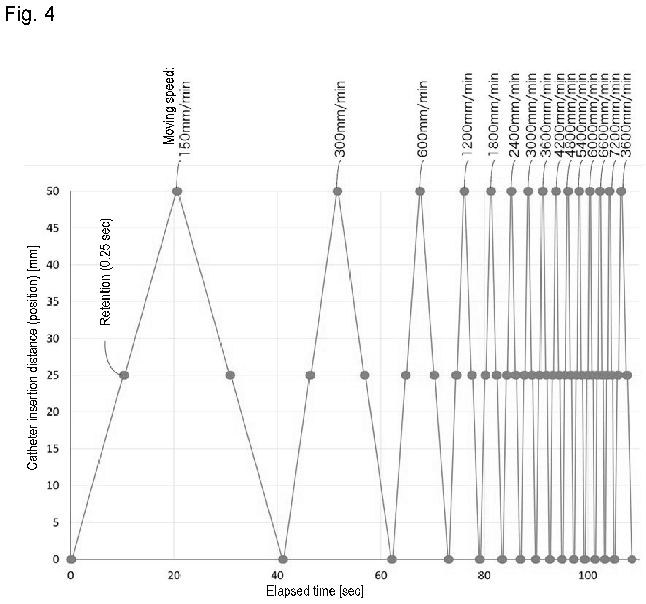

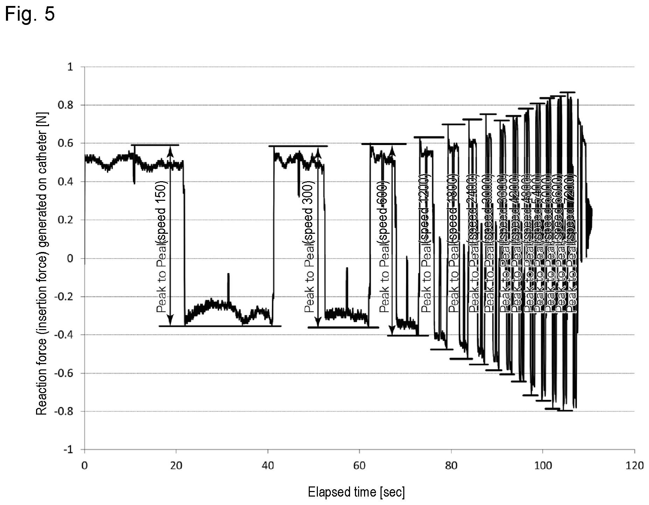

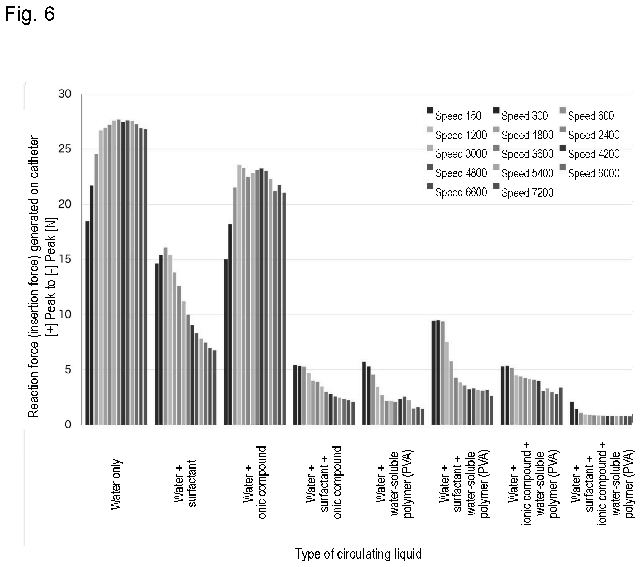

FIG. 5 illustrates the results measured by providing a rectangular wave-shaped reciprocation trajectory B illustrated in FIG. 4 in order to measure a reaction force (an insertion force) generated on the catheter during the reciprocation of the catheter. The reaction force hence can be compared with the speed of the reciprocation. FIG. 5 also describes a method of arranging measurement data in FIG. 6 and subsequent drawings. In the reciprocation trajectory B in FIG. 4, a retention for 0.25 second (a movement stop section) was set at each point indicated by a circle in order to measure presence or absence of adhesion and degree of occurrence thereof. Namely, reciprocation was set in a stepwise manner, such as (1) moving forward and moving forward again after the retention, (2) moving forward and moving backward after the retention, (3) moving backward and moving backward again after the retention, (4) moving backward and moving forward after the retention. Further, for the purpose of making it easy to identify a difference in the reaction force on a plurality of water-soluble polymers and the characteristics thereof, as illustrated in FIG. 5, a difference (a range described by Peak to Peak in FIG. 5) between a maximum value (a tensile force applied to the catheter) and a minimum value (a compressive force applied to the catheter) of the reaction force in each reciprocation cycle (a section in which forward movement and backward movement are combined) was extracted from measurement data for each moving speed from a speed of 150 mm/min to 7200 mm/min, and 14 numerical values in total were converted into a form of a set of numerical values.

FIG. 6 is a diagram in which the rectangular wave-shaped reciprocation trajectory B illustrated in FIG. 4 is given to the measurement apparatus 20 to perform the measurement, and the obtained measurement data is expressed by the method described with reference to FIG. 5. This drawing illustrates the reaction force (the insertion force) applied to the catheter for eight types of circulating liquids obtained with water only or by changing combinations of two or more of (1) the water, (2) the surfactant, (3) the water-soluble ionic compound, and (4) the water-soluble polymer (PVA). The reaction force illustrated in a bar graph having 14 lines as one set in FIG. 6 are the Peak to Peak values of the reaction force (a catheter insertion force) with respect to each moving speed described with reference to FIG. 5. For each set, the values of the reaction force at 150, 300, 600, 1200, 1800, 2400, 3000, 3600, 4200, 4800, 5400, 6000, 6600, and 7200 mm/min are illustrated in order from a bar graph on the left side.

From the measurement results illustrated in FIG. 6, as an overall tendency, the reaction force (the catheter insertion force) in a region where the moving speed is slow is larger than that in a region where the moving speed is fast. It can be confirmed that this tendency is suppressed by adding the ionic compound. Note that it can also be confirmed that the lubricity can not be obtained only with the ionic compound. In addition, it can also be confirmed that the lubricity obtained by the surfactant or the water-soluble polymer is enhanced by the addition of the ionic compound.

Further, from the results of FIG. 6, it can be confirmed that in the circulating liquid to which both the surfactant and the water-soluble polymer are added, a larger reaction force is generated than in the circulating liquid to which only the water-soluble polymer is added. Consequently it can be confirmed that not only a synergistic effect due to mixing and addition is not obtained, but also the lubricity of the water-soluble polymer is impaired by the mixing.

In the circulating liquid to which three of the surfactant, the ionic compound, and the water-soluble polymer are added, it can be confirmed that a large synergistic effect is generated in lubricity without impairing the respective properties.

Note that, in the measurement with the rectangular waver trajectory B (FIG. 4), unlike the sinusoidal wave trajectory A (FIG. 2), the toughness of the lubricating layer hardly appears as an explicit feature in the measurement results. Therefore, although there seems to be little difference in toughness, the toughness of the lubricating layer is significantly improved in a liquid to which the three are added as compared with other circulating liquids. This difference in toughness of the lubricating layer can be explicitly measured by a measurement method using the sinusoidal wave trajectory A (the dither in FIG. 2). In a case where the toughness is low, in a section where the amplitude of the sinusoidal wave trajectory A decreases, the lubricating layer collapses, the catheter and the inner peripheral surface of the blood vessel model come into direct contact with each other, and a markedly large reaction force is generated in the same section.

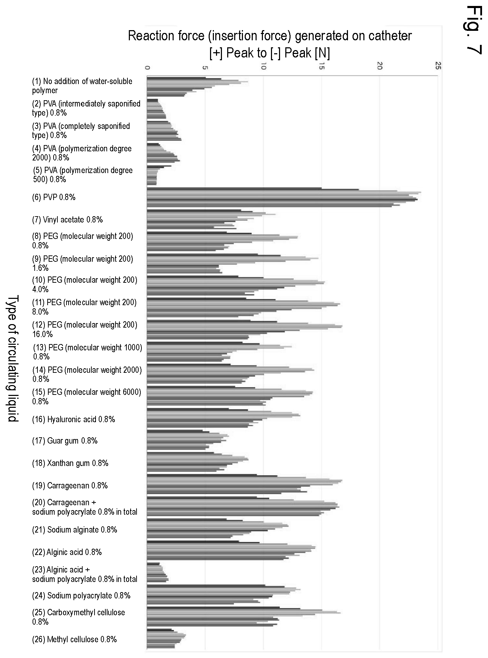

FIG. 7 illustrates the collated measurement results measured by the same measurement method as in FIG. 6 for the circulating liquids obtained by keeping the concentrations of the surfactant and the water-soluble ionic compound constant and changing the type of the water-soluble polymer.

In FIG. 7, No. 1 is a circulating liquid containing no water-soluble polymer but containing only the surfactant and the water-soluble ionic compound.

The water-soluble polymer blended in No. 2 is PVA (intermediately saponified type), and the added amount thereof is 0.8 wt%.

The water-soluble polymer blended in No. 3 is PVA (completely saponified type), and the added amount thereof is 0.8 wt%.

The water-soluble polymer blended in No. 4 is PVA (polymerization degree: 2000), and the added amount thereof is 0.8 wt%.

The water-soluble polymer blended in No. 5 is PVA (polymerization degree: 500), and the added amount thereof is 0.8 wt%.

The water-soluble polymer blended in No. 6 is PVP, and the added amount is 0.8 wt%.

The water-soluble polymer blended in No. 7 is vinyl acetate, and the added amount thereof is 0.8 wt%.

The water-soluble polymer blended in No. 8 is PEG (molecular weight: 200), and the added amount is 0.8 wt%.

The water-soluble polymer blended in No. 9 is PEG (molecular weight: 200), and the added amount thereof is 1.6 wt%.

The water-soluble polymer blended in No. 10 is PEG (molecular weight: 200), and the added amount thereof is 4.0 wt%.

The water-soluble polymer blended in No. 11 is PEG (molecular weight: 200), and the added amount thereof is 8.0 wt%.

The water-soluble polymer blended in No. 12 is PEG (molecular weight: 200), and the added amount thereof is 16.0 wt%.

The water-soluble polymer blended in No. 13 is PEG (molecular weight: 1000), and the added amount thereof is 0.8 wt%.

The water-soluble polymer blended in No. 14 is PEG (molecular weight: 2000), and the added amount thereof is 0.8 wt%.

The water-soluble polymer blended in No. 15 is PEG (molecular weight: 6000), and the added amount thereof is 0.8 wt%.

The water-soluble polymer blended in No. 16 is hyaluronic acid, and the added amount thereof is 0.8 wt%.

The water-soluble polymer blended in No. 17 is guar gum, and the added amount thereof is 0.8 wt%.

The water-soluble polymer blended in No. 18 is xanthan gum, and the added amount thereof is 0.8 wt%.

The water-soluble polymer blended in No. 19 is carrageenan, and the added amount thereof is 0.8 wt%.

The water-soluble polymer blended in No. 20 is carrageenan + sodium polyacrylate, and the added amount thereof is 0.8 wt% in total.

The water-soluble polymer blended in No. 21 is sodium alginate, and the added amount thereof is 0.8 wt%.

The water-soluble polymer blended in No. 22 is alginic acid, and the added amount thereof is 0.8 wt%.

The water-soluble polymer blended in No. 23 is alginic acid + sodium polyacrylate, and the added amount thereof is 0.8 wt%.

The water-soluble polymer blended in No. 24 is sodium polyacrylate, and the added amount thereof is 0.8 wt%.

The water-soluble polymer blended in No. 25 is carboxymethyl cellulose, and the added amount thereof is 0.8 wt%.

The water-soluble polymer blended in No. 26 is methyl cellulose, and the added amount thereof is 0.8 wt%.

The following can be confirmed from the measurement results illustrated in FIG. 7.

In a case of using the water-soluble polymer having the hydrophilic group and the hydrophobic group, such as the PVAs of No. 2 to No. 5 and the methyl cellulose of No. 26, the effects of the present invention are exhibited, and the lubricity is greatly improved. Note that, in this state, the toughness of the lubricating layer is also greatly improved in each of the circulating liquids. As confirmed from No. 3, since PVA used industrially is not completely saponified, even the PVA of a completely saponified type has both the hydrophilic group and the hydrophobic group, and exhibits the effects of the present invention singly.

On the other hand, in a case of using the water-soluble polymer having only the hydrophilic group or only the hydrophobic group (hydrophilicity or hydrophobicity resulting from molecular arrangement and the like) such as the water-soluble polymers of No. 6 to No. 22 as well as No. 24 and No. 25, it is clearly confirmed that the effects of the present invention are not exhibited at all. In particular, it can be confirmed that, in a case where the water-soluble polymer of one of No. 6 to No. 22 as well as No. 24 and No. 25 is added, the lubricity is significantly reduced as compared with a case (No. 1) without the addition thereof.

In addition, as confirmed from the result of No. 23, in a case where No. 22 (having only the hydrophobic group) and No. 24 (having only the hydrophilic group), which do not exhibit the effects of the present invention singly, are added in combination, it is confirmed that the effect of the present invention is remarkably exhibited in reversal.

In this description, the water-soluble polymer refers to a polymer that dissolves in water, and includes a polymer that exhibits water solubility in a state of being dissolved in a water-soluble solvent (such as a crosslinked product of the PVA and a polysaccharide dissolved therein), in addition to a polymer that dissolves as a simple substance as represented by the PVA. For example, the alginic acid of No. 22 has only the hydrophobic group, and thus is insoluble in water alone, but is dissolved in the water by being added together with the PVA being one of No. 2 to No. 5.

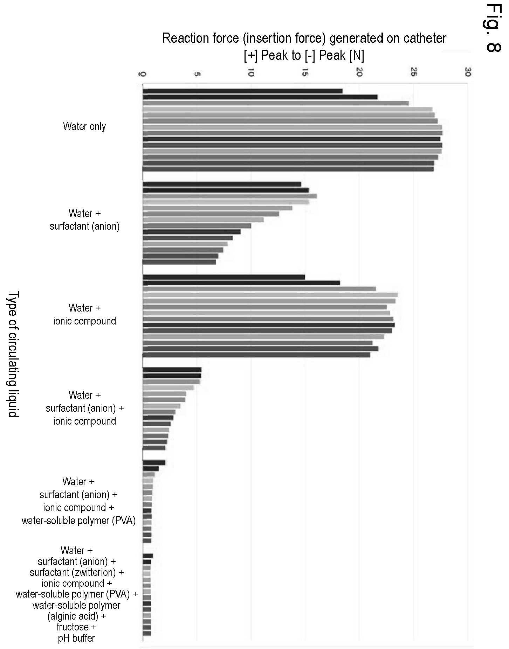

With the effects of the present invention, the lubricating property (including a relationship with the moving speed of the catheter), a tendency associated with zeta potential, and the like can be further finely adjusted by blending a plurality of types of surfactants, water-soluble ionic compounds, and water-soluble polymers in consideration of the type, material, and the like of the catheter to be used. Accordingly, it is possible to improve the functionality, or to prepare a circulating liquid specialized for a specific purpose or condition. FIG. 8 illustrates a case where measurement is performed by the same method as in FIG. 6 using a circulating liquid (on the rightmost side of FIG. 8) prepared for the purpose of keeping the reaction force applied to the catheter constant without depending on the moving speed of the catheter. In this example, an anionic surfactant (0.2 wt%) and a zwitterionic surfactant (0.12 wt%) are used in combination as the surfactant, and the PVA (0.32 wt%), the alginic acid (0.08 wt%), and the fructose (0.45 wt%) are used in combination as the water-soluble polymer. In addition, a pH buffer (0.02 wt%) is added for the purpose of making the tendency of the zeta potential close to that in blood of a living body and making a property exhibited by the catheter close to that in the human blood vessel.

In addition, from results of FIG. 8, it can be confirmed that the effects of the present invention are not lost by blending of a saccharide. In a case where the PVA is singly added as the water-soluble polymer, when the circulating liquid containing the PVA dries, the remaining PVA forms a film, and the film may clog or adhere to the inside of the blood vessel model and equipment such as the catheter and a pump, thereby interfering with subsequent use. If the saccharide coexists, a polymer film is not formed during the drying, and the circulating liquid becomes fine powders, so that easy cleaning such as wiping becomes possible.

In the circulating liquid, the entanglement state and the size of molecules may be changed by heating or cooling, and thus the lubricating property can be adjusted. For example, in the circulating liquid in which the PVA is blended, by heating it at a certain temperature (for example, 35°C) for a certain period (for example, 2 minutes), turbidity was removed and the transparency was increased, furthermore the lubricating property was significantly improved as compared with that before the heating. Even after the temperature of the circulating liquid from which the turbidity has been removed by the heating returned to a room temperature (about 25°C) again, excellent lubricity was irreversibly maintained together with high transparency.

When the water-soluble polymer such as the PVA is used, the pH of the circulating liquid is preferably maintained in a range of weak basicity to weak acidity in order to similarly provide an environment close to that of the blood. Therefore, the pH buffer can be blended in the circulating liquid as an auxiliary agent. In particular, in a case of using an acidic circulating liquid having a pH of less than 5.0, a hydrophilic coating covering the surface of the catheter was deactivated in a short time, causing adverse effects such as a significant decrease in the lubricity of the catheter.

According to the study of the present inventors, after the circulating liquid of each of examples containing the water-soluble polymer was brought into contact with the inner wall of the blood vessel model and the outer periphery of the sheath for the catheter, good lubricity was maintained between the blood vessel model and the catheter even using another circulating fluid (for example, one in which only the surfactant was blended). This is because a layer of the water-soluble polymer material is formed at least on the inner peripheral wall of the blood vessel model due to the circulating liquid of the example.

In other words, it can be considered that the circulating liquid of each of the examples forms a certain lubricating layer on the surface of the silicone rubber and a surface of a resin material constituting the sheath, and the lubricity is obtained by maintaining the layer. Note that a sequestering agent (a chelating agent) was effective for cleaning and removing the remaining lubricating layer. Edetate and the like can be used as the chelating agent.

In a case where the blood vessel model is made of silicone rubber, the lubricating function of the present invention is less likely to be sufficiently and effectively exhibited if many unreacted parts remain in siloxane molecules constituting the silicone rubber. Therefore, the effects of the present invention is further enhanced by accelerating the reaction by heating or the like to reduce the unreacted parts, furthermore, using a TMS-forming agent (a trimethylsilylating agent) or the like to chemically modify functional groups such as a hydroxyl group, a carboxyl group, and an amino group in the unreacted parts of the silicone rubber or the like.

Therefore, the present invention can be expanded as follows.
(1) A lubricant that imparts lubricity between a first member containing a crosslinked polymer material and a second member containing a crosslinked polymer material, the lubricant comprising:
   one or two or more selected from water, a surfactant, a water-soluble ionic compound, a first water-soluble polymer having a hydrophilic group and a hydrophobic group, a mixture of a second water-soluble polymer having a hydrophilic group and a third polymer having a hydrophobic group, and a mixture of the first water-soluble polymer and the third polymer.
   Here, there is a blood vessel model made of silicone rubber, urethane rubber, or the like as an example of the first member, and there is a sheath of a catheter made of polyethylene or the like as an example of the second member.
   This lubricant may not limited for a combination of the blood vessel model and the catheter at all, and improves lubricity between cross linkable polymer materials.
(2) The lubricant according to (1), in which the surfactant includes a zwitterionic surfactant.
(3) The lubricant according to (1) or (2), in which the water-soluble polymer includes polyvinyl alcohol.
(4) The lubricant according to (3), in which the water-soluble polymer is a crosslinked product of the polyvinyl alcohol and a polysaccharide.
(5) The lubricant according to (4), further comprising a monosaccharide.
(6) The lubricant according to (1), further comprising a pH adjusting agent.

Such lubricant is valuable as a surface coating agent for a catheter.

In other words,
(11) A lubricant to be applied to a surface of a sheath for a catheter, the lubricant comprising one or two or more selected from water, a surfactant, a water-soluble ionic compound, a first water-soluble polymer having both a hydrophilic group and a hydrophobic group, a mixture of a second water-soluble polymer having a hydrophilic group and a third polymer having a hydrophobic group, and a mixture of the first water-soluble polymer and the third polymer.
(12) The lubricant according to (11), in which the surfactant includes a zwitterionic surfactant.
(13) The lubricant according to (11) or (12), in which the water-soluble polymer includes polyvinyl alcohol.
(14) The lubricant according to (13), in which the water-soluble polymer is a crosslinked product of the polyvinyl alcohol and a polysaccharide.
(15) The lubricant according to (14), further comprising a monosaccharide.
(16) The lubricant according to (11), further comprising a pH adjusting agent.
(17) A catheter including a film formed of the lubricant according to any one of (11) to (16).

The measurement apparatus illustrated in FIG. 1 is a newly created apparatus for evaluating the lubricity between the blood vessel model and the catheter. This apparatus of the present invention can be understood as follows.

(21) A measurement apparatus that evaluates lubricity between a blood vessel model and a catheter, the apparatus comprising:
a clip portion that clips the blood vessel model via an elastic material in a state where the catheter is inserted into the blood vessel model;
a fixing portion that fixes the clip portion;
a catheter driver that moves the catheter in an axial direction of the blood vessel model at a predetermined rhythm; and
a resistance measuring portion that measures resistance applied to the catheter driver when the catheter is moved.

(22) The measurement apparatus according to (21), in which an elastic modulus of the elastic material is equal to an elastic modulus of a human tissue.

(23) The measurement apparatus according to (22), in which the elastic material contains a silicone gel.

(24) The measurement apparatus according to any one of (21) to (23), in which the catheter driver varies a moving speed of the catheter.

The present invention is not limited to the description of the above embodiment and examples of the present invention at all. Various modifications that can be easily conceived of by a person skilled in the art without departing from the scope of the claims are also included in the present invention.

I would like to express my heartfelt gratitude to my father, Moritaka Ikeda, who always enthusiastically and kindly supported me in every aspect of the process of the present invention, including the design, prototyping, and experiments of the measurement apparatus and the measurement method, so that I dedicate the present patent which has finally reached the filing stage to them.

### REFERENCE SIGNS LIST

- 1: Catheter
- 5: Blood vessel model
- 20: Measurement apparatus

## Claims

1. A water-based circulating liquid used in a catheter simulator, the circulating liquid comprising one or two or more selected from a first water-soluble polymer having both a hydrophilic group and a hydrophobic group, a mixture of a second water-soluble polymer having a hydrophilic group and a third polymer having a hydrophobic group, and a mixture of the first water-soluble polymer and the third polymer.

2. The circulating liquid according to claim 1, wherein the water-soluble polymer accounts for 0.5 to 8.0 mass% with respect to a water component of the circulating liquid.

3. The circulating liquid according to claim 1, wherein the first water-soluble polymer is one or two or more selected from polyvinyl alcohol (PVA), methyl cellulose, synthetic polymers such as polyacrylic acid, polyacrylamide, polyethylene oxide, poly(vinylpyrrolidone), polyvinylamide, and polyamine in addition to natural polymers such as protein, and starch.

4. The circulating liquid according to claim 1, wherein the first water-soluble polymer is PVA or methyl cellulose.

5. The circulating liquid according to claim 4, wherein the first water-soluble polymer is PVA.

6. The circulating liquid according to claim 5, wherein the PVA has a number average molecular weight of 500 to 2000 and a saponification degree of 75 or more.

7. The circulating liquid according to claim 5, further comprising a pH buffer.

8. The circulating liquid according to claim 5, further comprising a saccharide.

9. The circulating liquid according to claim 8, wherein 50 to 200 parts by mass of the saccharide is blended with respect to 100 parts by mass of the PVA.

10. The circulating liquid according to claim 1, wherein
a blending ratio of the second water-soluble polymer and the third polymer in the mixture of the second water-soluble polymer and the third polymer is 1 : 1.

11. The circulating liquid according to claim 1, wherein the second water-soluble polymer is sodium polyacrylate, and the third polymer is alginic acid.

12. The circulating liquid according to claim 1, further comprising a surfactant and a water-soluble ionic compound.

13. The circulating liquid according to claim 10, further comprising a pH buffer.

14. The circulating liquid according to claim 10, wherein the surfactant is a single zwitterionic surfactant, a surfactant obtained by adding a zwitterionic surfactant to a cationic surfactant, or a surfactant obtained by adding a zwitterionic surfactant to an anionic surfactant.

15. A lubricity adjusting agent to be blended in a water-based circulating liquid used in a catheter simulator, the lubricity adjusting agent comprising:
one or two or more selected from a first water-soluble polymer having both a hydrophilic group and a hydrophobic group, a mixture of a second water-soluble polymer having a hydrophilic group and a third polymer having a hydrophobic group, and a mixture of the first water-soluble polymer and the third polymer.

16. A lubricity adjusting agent set comprising:
a first aqueous solution containing the lubricity adjusting agent according to claim 13;
a second aqueous solution containing a surfactant; and
a third aqueous solution containing a water-soluble ionic compound.

17. A catheter simulation method comprising:
circulating the circulating liquid according to claim 1 in a blood vessel model; and
inserting a catheter into the blood vessel model.

18. The method according to claim 15, further comprising chemically modifying functional groups of silicone rubber before circulating the circulating liquid when the blood vessel model is formed of the silicone rubber.

19. A cleaning method comprising removing the catheter according to claim 15 from the blood vessel model, and then cleaning the catheter with a cleaning liquid containing a chelating agent.

20. A lubricity adjusting agent of an aqueous type that imparts lubricity between a first member containing a crosslinked polymer material and a second member containing a crosslinked polymer material, the lubricity adjusting agent comprising:
one or two or more selected from a first water-soluble polymer having both a hydrophilic group and a hydrophobic group, a mixture of a second water-soluble polymer having a hydrophilic group and a third polymer having a hydrophobic group, and a mixture of the first water-soluble polymer and the third polymer.

21. The lubricity adjusting agent according to claim 18, wherein the first member is a blood vessel model, and the second member is a sheath for a catheter.

22. An aqueous composition comprising:
water;
a surfactant;
a water-soluble ionic compound; and
one or two or more selected from a first water-soluble polymer having both a hydrophilic group and a hydrophobic group, a mixture of a second water-soluble polymer having a hydrophilic group and a third polymer having a hydrophobic group, and a mixture of the first water-soluble polymer and the third polymer.
